(19) Europäisches Patentamt
European Patent Office
Office européen des brevets

(11) **EP 2 574 918 B1**

(12) **EUROPÄISCHE PATENTSCHRIFT**

(45) Veröffentlichungstag und Bekanntmachung des Hinweises auf die Patenterteilung:
**10.12.2014 Patentblatt 2014/50**

(51) Int Cl.:
*G01N 33/22* [(2006.01)]     *G01F 1/68* [(2006.01)]
*F23N 5/18* [(2006.01)]     *G01N 9/36* [(2006.01)]

(21) Anmeldenummer: **11007858.1**

(22) Anmeldetag: **28.09.2011**

(54) **Mikrothermisches Verfahren und Sensor zur Bestimmung physikalischer Gaseigenschaften**

Microthermal method and sensor for determining physical gas properties

Procédé et capteur microthermiques pour la détermination de propriétés de gaz physiques

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.04.2013 Patentblatt 2013/14**

(73) Patentinhaber: **MEMS AG**
**5200 BRUGG (CH)**

(72) Erfinder:
• **Prêtre, Philippe**
**5405 Baden-Dättwil (CH)**
• **Kempe, Andreas**
**8049 Zürich (CH)**
• **Denzler, Roland**
**8484 Weisslingen (CH)**

(74) Vertreter: **Steiner, Peter**
**Patente + Marken**
**Untere Wolfackerstrasse 16**
**8280 Kreuzlingen (CH)**

(56) Entgegenhaltungen:
**EP-A1- 1 391 703    EP-A1- 1 947 450**
**EP-A2- 2 042 850    GB-A- 2 312 508**
**GB-A- 2 333 371    US-A- 5 311 447**

**Beschreibung**

Die Erfindung bezieht sich auf ein Verfahren und auf einen mikrothermischen Sensor zur Bestimmung physikalischer Gaseigenschaften von Gasgemischen. Unter physikalischen Gaseigenschaften werden insbesondere die Dichte, Wärmeleitfähigkeit, Wärmekapazität und Viskosität und die daraus korrelierbaren, brenntechnisch relevanten Größen wie der Energieinhalt des Gasgemisches, ausgedrückt durch den Brennwert, den Wobbe-Index, die Methanzahl, und/oder den Luftbedarf, verstanden.

Die Erfindung kann im Zusammenspiel mit geeigneten Gasdurchflussmesstechniken diese auf einfache und kostengünstige Weise zu Gasqualitätssensoren hoher Genauigkeit aufwerten, wie sie sonst nicht oder nur in aufwändiger Art realisierbar sind für alle Prozesse, bei denen nebst den Gaseigenschaften gleichzeitig auch die durchgeflossenen Menge Gas von Interesse ist wie beispielsweise bei der Steuerung von Verbrennungsprozessen (Gasfeuerungsregelung), der Motorregelung von (Erd-)Gasmotoren oder der Luftbedarfsbestimmung und Leistungsregelung in Brennstoffzellen.

Bei Gasfeuerungsregelungen ist es wichtig, bei wechselnder Gasqualität die Brennerbelastung konstant zu halten. Der entsprechende Index zur Anzeige der Austauschbarkeit von Gasen ist der Wobbe Index, gebildet aus dem Brennwert und dem Dichteverhältnis zwischen Luft und diesem Gas. Bei gleichem Wobbe Index ergibt sich dann die gleiche Wärmebelastung eines Brenners.

Bei der Regelung von (Erd-)Gasmotoren ist zur Leistungs- bzw. Effizienzsteigerung die Kenntnis des Brennwerts bei wechselnden Gasqualitäten wichtig, zur Beurteilung des Zündverhaltens (Klopfen bzw. Zündaussetzer) wird hingegen bei Gas die zur Oktanzahl bei Benzin analoge Methanzahl herangezogen.

Für einen optimalen Verbrennungsprozess ist das richtige Mischverhältnis zwischen Brenngas und Luft wichtig, der sogenannte Luftbedarf. Bei zuwenig Luft bildet sich normalerweise Ruß (Abgase), was v.a. bei Brennstoffzellen zu deren Zerstörung führen kann. Zuviel Luft bei der Verbrennung resultiert in einer kleineren Leistungsausbeute. Dabei hängt der optimale Wert von der jeweiligen Anwendung ab, ändert sich aber wieder bei wechselnder Gasqualität. Es besteht eine sehr gute Korrelation zwischen Luftbedarf und Brennwert eines Gases.

Unter Gasdurchflussmesstechniken werden alle Messtechniken verstanden, die entweder den Volumen- oder den Massenfluss eines Gases unabhängig von dessen Eigenschaften bestimmen. Hierzu zählen beispielsweise Coriolismeter, die physikalisch inhärent den Massenfluss oder die Dichte (und damit den Volumenfluss) messen, während andere Messtechniken wie beispielsweise thermische Durchflusszähler diese Aufgabe nur bedingt lösen, da das Messprinzip inhärent von den Gaseigenschaften selbst abhängt.

Umgekehrt liefert die Erfindung ein Verfahren und einen mikrothermischen Sensor zur Bestimmung physikalischer Gaseigenschaften, bei denen die Vorgabe des Durchflusses durch das Ausnützen gasunabhängiger Strömungsdynamiken erreicht wird, beispielsweise mittels einer Taylor-Couette-Strömung in rotierenden Zylindern (Herbert Oertl (jr) (Hrsg.): Prandtl Führer durch die Strömungslehre 10., vollständig bearbeitete Auflage, Friedr. Vieweg & Sohn, Braunschweig/Wiesbaden 2001, ISBN 3-528-38209-0)

Die Idee der Erfindung ist die Bestimmung physikalischer Gaseigenschaften und die daraus korrelierbaren, brenntechnisch relevanten Größen eines Erdgasgemisches basierend auf einer mikrothermischen Messung mit Gasfluss, wobei die mit dieser Methode bestimmbaren Gaseigenschaften und die damit erreichbare Messgenauigkeit davon abhängen, ob und welche weiteren Gaseigenschaftsgrössen bekannt sind. Ausgegangen wird dabei von der das mikrothermische System beschreibenden, eindimensionalen Wärmeleitungsgleichung (Kerson Huang: Statistical Mechanics, 2.Auflage, John Wiley & Sons, New York 1987, ISBN 0-471-85913-3)

$$\frac{c_p}{\lambda} \cdot \rho \, \mathrm{v}_x \cdot \frac{d}{dx} T = \nabla^2 T + \frac{1}{\lambda} \Theta \; , \qquad\qquad (1)$$

wobei

$\mathrm{V}_x$   die Komponente der mittleren Fliessgeschwindigkeit (Geschwindigkeitsvektor) $\bar{\mathrm{v}}$ in x-Richtung, d. h. entlang des Gasstromes,

T   die Temperatur,

$\dfrac{d}{dx} T$   der Temperaturgradient,

$c_p$   die Wärmekapazität des Gases bei konstantem Druck,

$\rho$   die Dichte,

$\lambda$   die Wärmeleitfähigkeit des Gases,

$\Delta^2 T$      der Laplace-Operator, angewandt auf die Temperatur T, wobei

$$\nabla^2 = \left(\frac{d}{d_x}\right)^2 + \left(\frac{d}{dy}\right)^2 + \left(\frac{d}{dz}\right)^2$$

bedeuten. Da das Gas (Gasstrom) nur in x-Richtung fließt, werden die Komponenten $v_y$ bzw. $v_z$ in y-Richtung bzw. in z-Richtung der mittleren Fliessgeschwindigkeit $\bar{v}$ zu Null angenommen. $\Theta$ mit der Einheit Watt/m$^3$ beschreibt den Quellterm des Heizelements. Der Quellterm rührt bei der mikrothermischen Methode vom Heizdraht eines miniaturisierten, integrierten Hitzdrahtanemometers her, der Wärmeenergie in das System speist. Aus Gleichung (1) wird ersichtlich, dass dem Volumen- bzw. Massenfluss $v_x$ bzw. $\rho \cdot v_x$, der Dichte $\rho$, der Wärmekapazität $c_p$ und der Wärmeleitfähigkeit $\lambda$ im Vorfaktor

$$\frac{c_p}{\lambda} \cdot \rho \cdot v_x \qquad\qquad\qquad (2)$$

eine gleichwertige Rolle bei der Lösung der Gleichung zukommt, wobei die Wärmeleitfähigkeit $\lambda$ wegen des Quellterms $\Theta$ zusätzlich separat auf die Lösung der Gleichung (1) einwirkt. Die Umrechnung von mittlerer Fliessgeschwindigkeit in Volumenfluss ist dabei durch die (Querschnitts-)Geometrie der das Gas zuführenden Messleitung gegeben.

Ist nun als weitere Grösse z.B. der Massenfluss $\rho \cdot v_x$ bekannt und kann die Wärmeleitfähigkeit $\lambda$ des Gases separat gemessen werden, lässt sich auf das Verhältnis $c_p/\lambda$ und folglich auf die Wärmekapazität $c_p$ des Gases schließen, die Ausgangspunkt für die korrelierbaren, brenntechnisch relevanten Größen des Gasgemisches ist. In der Praxis ist deshalb v.a. die Variante eines bekannten Massenflusses von Interesse, insbesondere, wenn der Massenfluss aus einer Zusatzmessung unabhängig von der Gasart bestimmt werden kann. Gleiches gilt in umgekehrter Richtung: bei bekannter Wärmekapazität $c_p$ und Wärmeleitfähigkeit $\lambda$ des Gases kann der Massenfluss $\rho \cdot v_x$ bestimmt werden.

Dies lässt sich soweit verallgemeinern, als dass beim Bekanntsein einer dieser Grössen $\rho$, $v_x$, $c_p$ oder $\lambda$ oder einer Kombination aus einer dieser Grössen die komplementäre Kombination der jeweils anderen Grössen bestimmt werden kann, wenn die Wärmeleitfähigkeit $\lambda$ separat messbar ist.

Die mikrothermische Wärmeleitfähigkeitsmessung basiert auf einem integrierten CMOS Hitzdrahtanemometer. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen. Sie unterscheidet sich insbesondere von herkömmlichen, thermischen Massenflussmessern durch eine Messung direkt im Gasstrom und nicht von aussen an einer den Gasstrom umfassenden Metallkapillare.

Aus EP 2 015 056 B1 ist ein thermischer Durchflusssensor zur Bestimmung einer brenntechnisch relevanten Grösse bekannt, basierend auf einer Wärmeleitfähigkeitsmessung bei quasi bekanntem Massenfluss. Da eine kritische Düse zur Durchflussregelung benützt wird, kann damit nur ein konstanter Betriebsvolumenstrom im engsten Düsenquerschnitt erreicht werden, was nur eine ungenaue Bestimmung der Wärmekapazität $c_p$ des Gases zulässt.

Aus US7188519 sind eine Methode und ein mikrothermischer Sensor zur Bestimmung eines Flusses und der Wärmeleitfähigkeit eines Fluids bekannt. Allerdings ist dies gemäss Gleichung (1) nicht möglich ohne die genaue Kenntnis der Wärmekapazität des Fluids. Aus dem gleichen Grund werden alle thermischen wie auch mikrothermischen Massenflussmeter, wie beispielsweise in EP1 119 744 beschrieben, ihrem Namen nur gerecht, wenn zusätzlich die Wärmekapazität des Fluids bekannt ist.

Aus US 5 311 447 ist ein Verfahren zur Bestimmung der Gasqualität anhand von Korrelationen mit leicht meßbaren Gasparametern bekannt.

Aus EP1 155 690 ist ein Sensor zur Bestimmung brenntechnisch relevanter Grössen bekannt, bei dem der Massenfluss konstant gehalten wird und mittels einer thermischen Messung eine zur Wärmekapazität proportionale Grösse ermittelt wird. Da es sich nicht um einen mikrothermischen Sensor handelt, kann nicht auf die Wärmeleitfähigkeit geschlossen werden, womit die Bestimmung der Wärmekapazität und der daraus abgeleiteten, brenntechnisch relevanten Grössen nur bis auf einen Proportionalitätsfaktor möglich ist, was eine zusätzliche Kalibrierung mit bekannten Gaszusammensetzungen notwendig macht. Zudem entfällt die Information über die Wärmeleitfähigkeit und die Möglichkeit der Korrelation von $\lambda$ mit einer der brenntechnisch relevanten Grössen. Im Weiteren ist die Genauigkeit dieser Methode limitiert auf die trotzdem auftretenden Änderungen der nicht zugänglichen Wärmeleitfähigkeit $\lambda$. Als weiterer

Nachteil des Sensors erweist sich die Grösse des thermischen Sensors, sodass keine kostengünstige Aufwertung eines Sensors für eine der weiteren, einflussbestimmenden Grössen ($v_x$, $\rho$, $c_p$ und $\lambda$) zu einem Gasqualitätssensoren hoher Genauigkeit möglich ist.

Der Erfindung liegt daher die Aufgabe zugrunde, ein Verfahren zur einfachen und kostengünstigen Aufwertung geeigneten Gasdurchflussmesstechniken zu Gasqualitätssensoren hoher Genauigkeit anzugeben, die brenntechnisch relevante Größen wie den Brennwert, den Wobbe-Index, die Methanzahl und/oder den Luftbedarf über Korreiationen mit den Grundeigenschaften Wärmekapazität, Wärmeleitfähigkeit oder Dichte eines Gases bestimmen. Des Weiteren soll ein nach dem erfindungsgemäßen Verfahren arbeitender Sensor angegeben werden.

Die Aufgabe wird bezüglich des Verfahrens gelöst durch ein Verfahren nach Anspruch 1.

Bezüglich des Sensors wird die Aufgabe gelöst durch einen Sensor nach Anspruch 8.

Die Erfindung wird nachstehend anhand der in der Zeichnung dargestellten Ausführungsbeispielen erläutert. Es zeigen:

Fig. 1        die H- / L-Gas Unterscheidung bei bekannter Wärmediffusivität und -leitfähigkeit ,

Fig. 2        das Prinzip der komplementären Grössen des mikrothermischen Messverfahrens,

Fig. 3        das Schema des Sensorblocks mit integriertem, mikrothermischen CMOS-Hitzdrahtanemometer,

Fig. 4a,b     Ausführungsbeispiele für den schematischen Aufbau eines Sensors zur Gasqualitätsbestimmung,

Fig. 5a,b     Ausführungsbeispiele für den schematischen Aufbau eines Sensors zur Gasqualitätsbestimmung in einer von der Gashauptleitung wegführenden Messleitung,

Fig. 6a,b,c   Ausführungsbeispiele zur Erzeugung einer gasunabhängigen Strömungsdynamik (Taylor-Couette-Strömung).

Mit anderen Worten wird die Aufgabe bezüglich des Verfahrens erfindungsgemäß gelöst durch eine Bestimmung der Dichte $\rho$, der Wärmekapazität $c_p$, der Wärmeleitfähigkeit $\lambda$ oder einer Kombination dieser Grössen zusammen mit der mikrothermische Messung der im Produkt von Gleichung (2) komplementären Grösse, je nachdem, welche Einzelgrösse oder Kombination von Grössen den besten Ausgangspunkt zur Bestimmung der brenntechnisch relevanten Größen bildet.

Gemäß einer vorteilhaften Ausführungsform der Erfindung wird mit dem mikrothermischen Sensor ein Massenflussmeter zum Gasqualitätssensor aufgewertet, indem das Verhältnis zwischen Wärmekapazität und Wärmeleitfähigkeit $c_p/\lambda$ als komplementäre Grösse zum Massenfluss $\rho \cdot v_x$ in Gleichung (2) gemessen wird. Mit der, der mikrothermischen Methode eigen zu nennenden Möglichkeit der separaten Bestimmung der Wärmeleitfähigkeit $\lambda$ lässt sich sodann die Wärmekapazität $c_p$ als Ausgangspunkt zur Korrelation des Brennwerts eines Gasgemisches bestimmen.

Gemäß einer weiteren, vorteilhaften Ausführungsform der Erfindung wird mit dem mikrothermischen Sensor ein gleichzeitiges Massen- und Volumenflussmeter zum Gasqualitätssensor aufgewertet, indem das Verhältnis zwischen Wärmekapazität und Wärmeleitfähigkeit $c_p/\lambda$ als komplementäre Grösse zum Massenfluss $\rho \cdot v_x$ in Gleichung (2) gemessen wird. Mit der, der mikrothermischen Methode eigen zu nennenden Möglichkeit der separaten Bestimmung der Wärmeleitfähigkeit $\lambda$ lässt sich sodann die Wärmekapazität $c_p$ als Ausgangspunkt zur Korrelation des Brennwerts eines Gasgemisches bestimmen, welcher zusammen mit der Dichte aus dem Verhältnis von Massen- zu Volumenfluss das Berechnen des Wobbe-Index zulässt.

Gasgemische in Erdgasanwendungen werden normalerweise in H-Gase ("High Calorific Value", z. B. Erdgas aus Russland und der Nordsee) und L-Gase ("Low Calorific Value", z. B. Erdgas aus Holland) unterteilt, wobei die Unterteilung definitionsgemäß bei einem Brennwert von ca. 10 kWh/m$^3$ bzw. einem Wobbe-Index von ca. 13 kWh/m$^3$ erfolgt (für eine Temperatur von 0°C und einen Druck von 1013.25 mbar). Die Unterteilung ist sowohl technisch (Gasbrenner) als auch administrativ (Abrechnungswesen) wichtig, kann gemäss den genannten Kriterien jedoch nur erfolgen, wenn letztere bekannt sind.

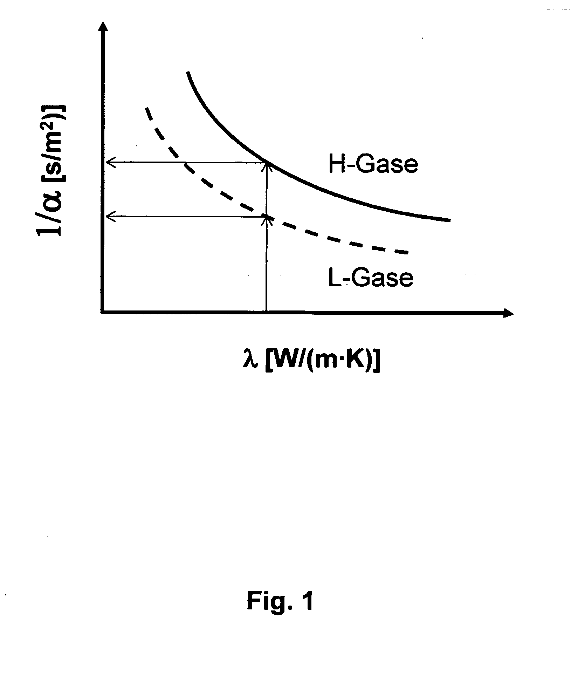

Gemäß einer weiteren, vorteilhaften Ausführungsform der Erfindung wird mit dem mikrothermischen Sensor ein Volumenflussmeter zum Gasquaiitätssensor aufgewertet, indem der Kehrwert der Wärmediffusivität, $1/\alpha = c_p \cdot \rho/\lambda$, als komplementäre Grösse zum Volumenfluss $v_x$ in Gleichung (2) gemessen wird. Mit der, der mikrothermischen Methode eigen zu nennenden Möglichkeit der separaten Bestimmung der Wärmeleitfähigkeit $\lambda$ lässt sich gemäss Fig. 1 die Wärmediffusivität $\alpha$ als Funktion von $\lambda$ zur Unterscheidung zwischen H- und L-Gas heranziehen, was Gasverbraucher und Gasmessgeräte sich automatisch auf H- oder L-Gas einstellen lässt.

Gemäß einer weiteren, vorteilhaften Ausführungsform der Erfindung wird mit dem mikrothermischen Sensor ein Dichtesensor zum Gasqualitätssensor aufgewertet, indem das Produkt aus dem Verhältnis zwischen Wärmekapazität und Wärmeleitfähigkeit mit dem Volumenfluss, $c_p/\lambda \cdot v_x$, als komplementäre Grösse zur Dichte $\rho$ in Gleichung (2) gemessen wird. Mit der, der mikrothermischen Methode eigen zu nennenden Möglichkeit der separaten Bestimmung der Wärmeleitfähigkeit $\lambda$ lässt sich sodann der "Wärmekapazitätsfluss" $c_p.v_x$ als Ausgangspunkt zur Korrelation des Brennwert-, sprich Energieflusses eines Gasgemisches bestimmen.

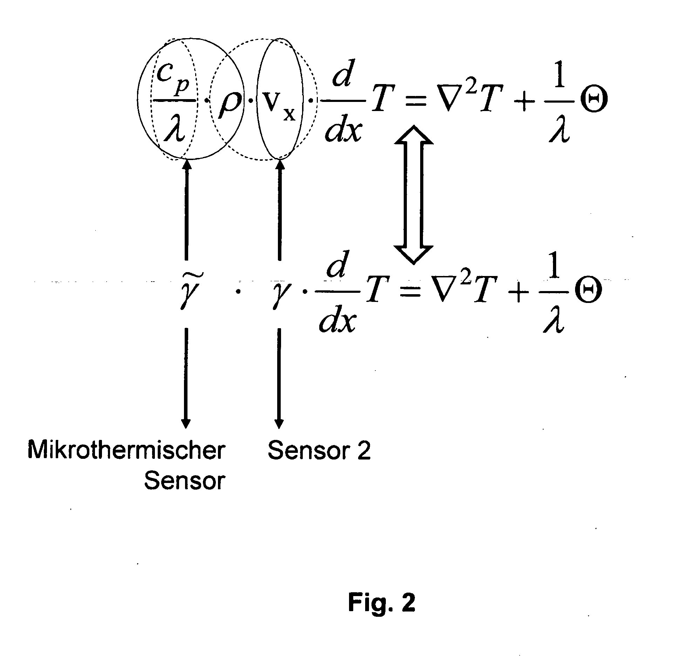

Gemäß einer allgemeinen Ausführungsform der Erfindung wird, wie in Fig. 2 gezeigt, mit dem mikrothermischen Sensor jeglicher Sensor für eine Grösse $\gamma$, sei dies die Dichte $\rho$, Wärmekapazität $c_p$, der Massen- oder Volumenfluss oder eine Kombination dieser Grössen, zum Gasqualitätssensor aufgewertet, indem mit dem mikrothermischen Sensor die zu $\gamma$ komplementäre Grösse $\tilde{\gamma}$

$$\tilde{\gamma} = \left( \frac{c_p}{\lambda} \cdot \rho \, \mathbf{v_x} \right) \Big/ \gamma \qquad\qquad (3)$$

und dazu separat die Wärmeleitfähigkeit $\lambda$ gemessen und diese Information zur Bestimmung brenntechnisch relevanten Größen eines Gasgemisches, ausgedrückt durch den Brennwert, den Wobbe-Index, die Methanzahl, und/oder den Luftbedarf benützt wird.

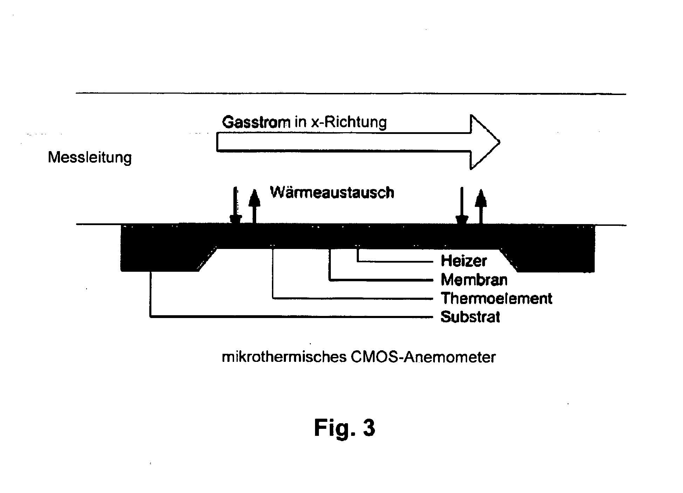

Ein erfindungsgemäßer, mikrothermischer Sensor zur Bestimmung physikalischer Gaseigenschaften, insbesondere eines Gasgemisches, arbeitet nach dem erfindungsgemäßen Verfahren mit einem Sensorblock mit integriertem, mikrothermischen CMOS-Hitzdrahtanemometer, über welches das Gasgemisch fliesst, durch Messen zweier Temperaturen stromauf- und stromabwärts zum Heizdraht, wie in Fig. 3 gezeigt. Die Temperaturinformation aus stromauf- und stromabwärts liegendem Temperaturfühler erlaubt es mit Hilfe der Lösung der Gleichung (1) die Grösse in Gleichung (2) und separat die Wärmeleitfähigkeit des Gasgemisches zu bestimmen.

Gemäß einer vorteilhaften Ausführungsform der Erfindung wird das integrierte CMOS-Hitzdrahtanemometer zusammen mit einem Coriolismeter verwendet, wobei der Sensorblock mit CMOS-Hitzdrahtanemometer in der Messleitung des Coriolismeters integriert ist und aus der Massen- bzw. Volumenflussinformation des Coriolismeters die Grösse

$$\tilde{\gamma} = \frac{c_p}{\lambda} \qquad\qquad (4)$$

und daraus zusammen mit der Information der Wärmeleitfähigkeit $\lambda$ die Wärmekapazität $c_p$ bestimmt wird, welche als Ausgangspunkt für die Korrelation des Brennwerts und zusammen mit der Dichte des Wobbe-Index dient.

Gemäß einer weiteren, vorteilhaften Ausführungsform der Erfindung wird der Sensorblock mit CMOS-Hitzdrahtanemometer in einer Anordnung mit gasunabhängiger Strömungsdynamik eingebettet, bei der der Volumenfluss vorgegeben und mit dem CMOS-Hitzdrahtanemometer der Kehrwert der Wärmediffusivität

$$\tilde{\gamma} = 1/\alpha = \frac{c_p}{\lambda} \cdot \rho \qquad\qquad (5)$$

bestimmt wird, was zusammen mit der Information der Wärmeleitfähigkeit $\lambda$ eine Unterscheidung von H- und L-Gasen ermöglicht, damit sich Gasverbraucher und Gasmessgeräte automatisch auf H- oder L-Gas einstellen können.

Gemäß einer vorteilhaften Ausführungsform der Erfindung ist die gasunabhängige Strömungsdynamik als Taylor-Couette-Strömung zwischen zwei relativ zueinander bewegten Platten oder relativ zueinander rotierenden Scheiben oder Zylindern ausgebildet.

Gemäß einer weiteren, vorteilhaften Ausführungsform der Erfindung wird das integrierte CMOS-Hitzdrahtanemometer zusammen mit einem Dichtesensor verwendet, wobei der Dichtesensor direkt im Sensorblock mit CMOS-Hitzdrahtanemometer integriert sein kann und aus der Dichteinformation die Grösse

$$\widetilde{\gamma} = \frac{c_p}{\lambda} \cdot v_x \qquad\qquad (6)$$

und daraus zusammen mit der Information der Wärmeleitfähigkeit λ der "Wärmekapazitätsfluss" $c_p \cdot v_x$ bestimmt werden kann, welcher als Ausgangspunkt für die Korrelation des Energieflusses dient.

Die mit der Erfindung erzielbaren Vorteile bestehen insbesondere darin, dass Messgeräte für physikalische Gaseigenschaften wie Dichte, Wärmekapazität, Massen- oder Volumenfluss auf einfache und kostengünstige Weise mit einem nach dem erfindungsgemäßen Verfahren arbeitenden Sensor mit integriertem CMOS-Hitzdrahtanemometer zu einem Gasqualitätssensor hoher Genauigkeit für brenntechnisch relevante Grössen wie Brennwert, Wobbe-Index, Methanzahl oder Luftbedarf aufgewertet werden können, um Massenmarktanwendungen zu ermöglichen.

Weitere Vorteile sind aus der nachstehenden Beschreibung ersichtlich.

Vorteilhafte Ausgestaltungen der Erfindung sind in den Unteransprüchen gekennzeichnet.

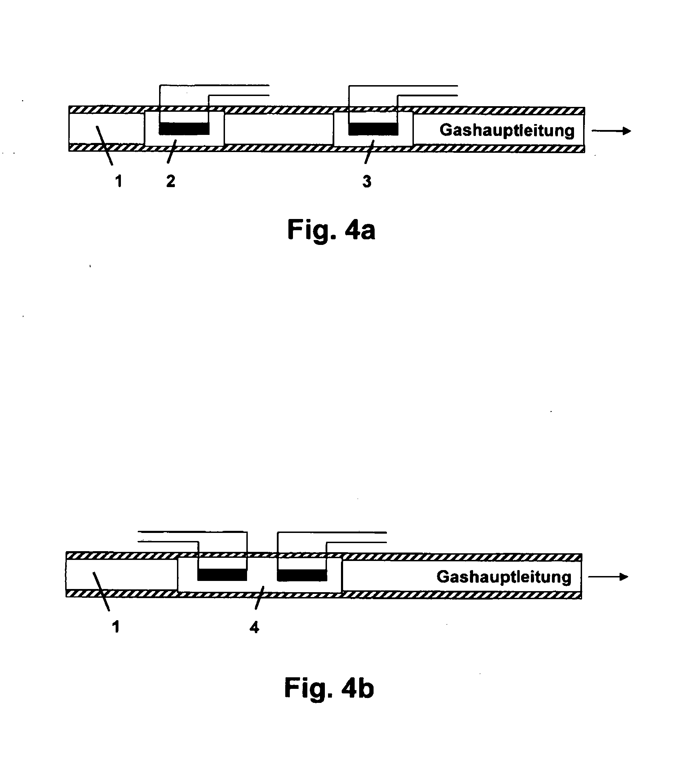

Ein einfacher Aufbau eines erfindungsgemässen Sensors zur Messung brenntechnisch relevanter Grössen von Gasen ist in Fig. 4a gezeigt. Gas aus der Gashauptleitung 1, welche z.B. die Zufuhrleitung zum Motor eines erdgasbetriebenen Fahrzeugs ist, fliesst zuerst durch den Sensor 2 für eine oder eine Kombination der physikalischen Gaseigenschaften Dichte, Wärmekapazität, Wärmeleitfähigkeit, Massen- oder Volumenfluss und danach durch den Sensorblock 3 des mikrothermischen Sensors mit integriertem CMOS-Hitzdrahtanemometer. Alternativ können die beiden Sensoren wie in Fig. 4b im gleichen Sensorblock 4 untergebracht sein.

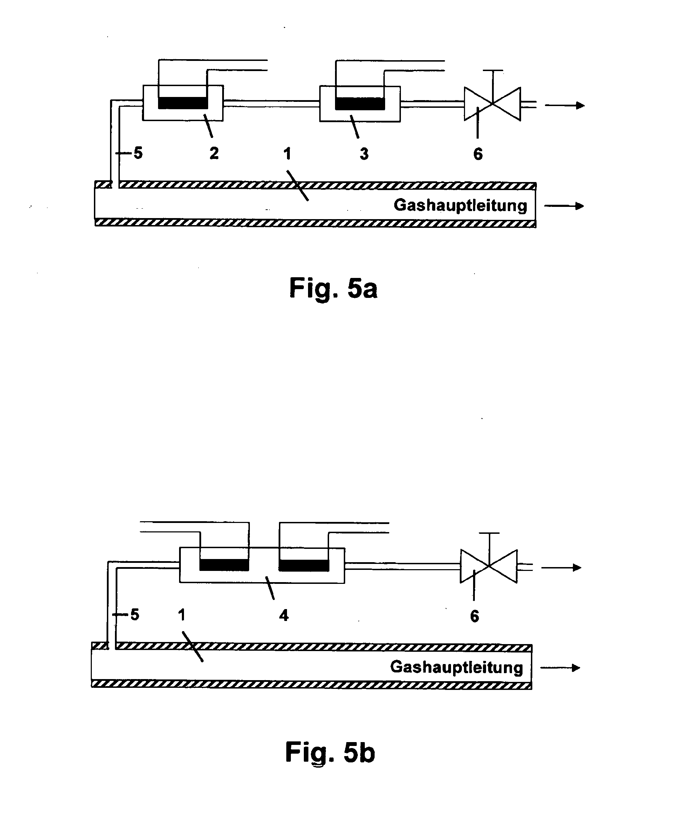

Ein weiterer, einfacher Aufbau eines erfindungsgemässen Sensors zur Messung brenntechnisch relevanter Grössen von Gasen ist in Fig. 5a gezeigt. Gas aus der Gashauptleitung 1 wird über eine Messleitung 5 zum Sensor 2 für eine physikalische Gaseigenschaft Dichte, Wärmekapazität, Wärmeleitfähigkeit, Massen- oder Volumenfluss oder eine Kombination daraus geführt, um danach den Sensorblock des mikrothermischen Sensors mit integriertem CMOS-Hitzdrahtanemometer zu durchfliessen. Alternativ können die beiden Sensoren wie in Fig. 5b im gleichen Sensorblock 4 untergebracht sein. Ein Absperrventil 6 am Auslass des Sensors 3 bzw. 4 dient dazu, den Gasfluss zu unterbrechen bzw. sich in Intervallen zur Spülung der Messleitung und für den Messvorgang zu öffnen.

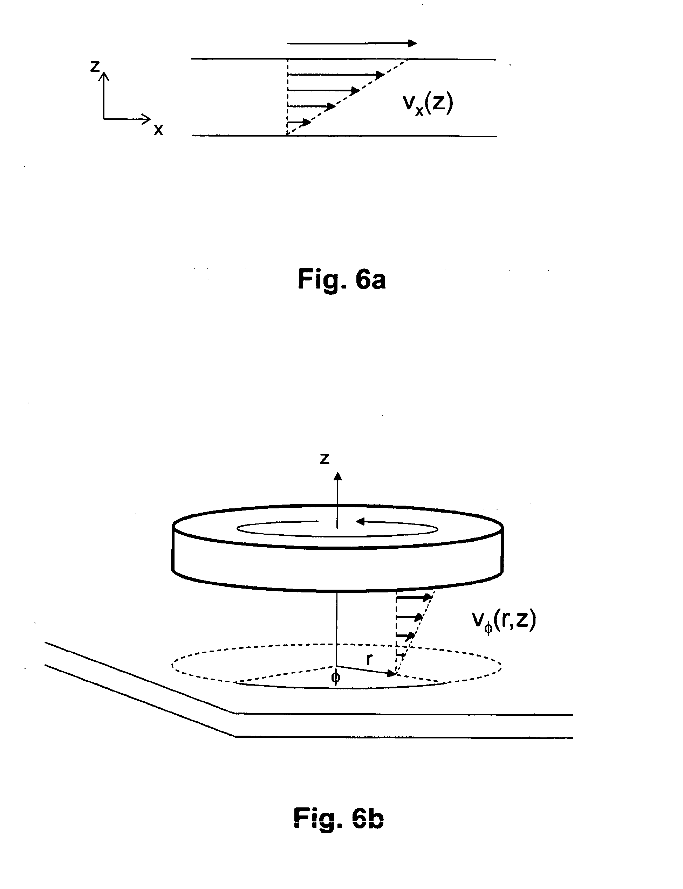

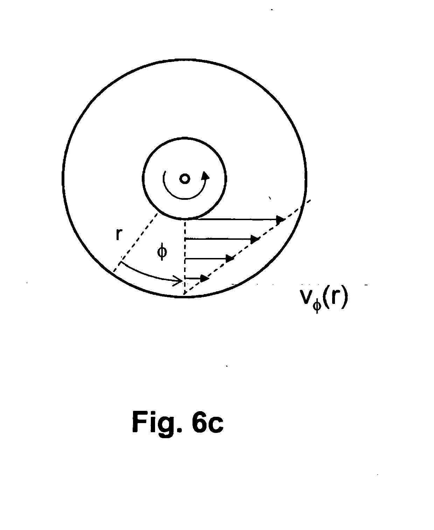

Ein einfacher Aufbau zur Erzeugung einer gasunabhängigen Strömungsdynamik ist in Fig. 6a gezeigt. Zwischen zwei sich relativ zueinander bewegenden Platten baut sich ein Strömungsprofil mit wohldefinierter Strömungsgeschwindigkeit $v_x$ in Richtung der Relativbewegung auf, in welches der mikrothermische Sensor mit integriertem CMOS-Hitzdrahtanemometer plaziert werden kann. Alternativ kann wie in Fig. 6b ein ähnliches Strömungsprofil mit relativ zu einander rotierenden Scheiben erzeugt werden, mit einer wohldefinierten Strömungsgeschwindigkeit in Drehrichtung φ. Als weitere Alternative sind zwei relativ zueinander rotierende Zylinder in Fig. 6c gezeigt, ebenfalls mit einer wohldefinierten, radial abhängigen Strömungsgeschwindigkeit in Drehrichtung φ.

Bezugszeichenliste:

| | |
|---|---|
| 1 | Gashauptleitung |
| 2 | Sensor zur Messung einer physikalischen Gaseigenschaft |
| 3 | dazu komplementär messender, mikrothermischer Sensor |
| 4 | Kombigehäuse mit Sensor 2 und 3 |
| 5 | Messleitung |
| 6 | Absperrventil |

| | |
|---|---|
| γ | physikalische Gaseigenschaft |
| $\widetilde{\gamma}$ | zu γ komplementäre, physikalische Gaseigenschaft |
| $c_p$ | Wärmekapazität |
| T | Temperatur |
| λ | Wärmeleitfähigkeit |
| α | Wärmediffusivität |
| ρ | Dichte |
| Θ | Quellterm des Heizelements |
| ∇ | Laplace-Operator |
| φ | Drehrichtung der Taylor-Couette Strömung |
| r | Radialvariable der Taylor-Couette Strömung |

**Patentansprüche**

1. Verfahren zur Bestimmung physikalischer Gaseigenschaften von Gasgemischen zur Korrelation brenntechnisch relevanter Größen der Gasgemische mittels Kombination eines ersten Sensors für die physikalische Gaseigenschaft ($\gamma$), beispielsweise die Dichte ($\rho$) oder die Wärmekapazität ($c_p$) oder den Massen- oder Volumenfluss oder eine Kombination dieser Größen, mit einem mikrothermischen Sensor zur Messung der dazu komplementären Grösse $\tilde{\gamma}$ in Bezug auf das Verhältnis ($v_x \cdot (\rho \cdot c_p/\lambda)$) zwischen Fliessgeschwindigkeit ($v_x$) und Wärmediffusivität ($\lambda/(\rho \cdot c_p)$) des Gasgemisches, wobei $\gamma \cdot \tilde{\gamma} = v_x \cdot (\rho \cdot c_p/\lambda)$ ist, **dadurch gekennzeichnet, dass** mindestens der mikrothermische Sensor mit einem Gasfluss beaufschlagt und die Wärmeleitfähigkeit ($\lambda$) des Gasgemisches mit dem mikrothermischen Sensor separat gemessen wird.

2. Verfahren nach Anspruch 1, **dadurch gekennzeichnet, dass** die physikalische Gaseigenschaft $\gamma$ die Dichte, die Wärmekapazität, der Massen- oder Volumenfluss oder eine Kombination dieser Grössen und die brenntechnisch relevante Größe der Brennwert, der Wobbe-Index, die Methanzahl und/oder der Luftbedarf ist.

3. Verfahren nach Anspruch 1, **dadurch gekennzeichnet, dass** die physikalische Gaseigenschaft ($\gamma$) der Volumenfluss ($v_x \cdot A$) eines Gasgemisches ist, womit die durch den mikrothermischen Sensor bestimmte Grösse ($\tilde{\gamma}$) das Produkt (($1/A) \cdot (\rho \cdot c_p/\lambda)$) aus dem Kehrwert ($1/A$) des Durchflussquerschnitts mit dem Kehrwert ($\rho \cdot c_p/\lambda$) der Wärmediffusivität eines Gasgemisches ist, was zusammen mit Kenntnis der Wärmeleitfähigkeit ($\lambda$) eine Unterscheidung in H- und L-Gase ermöglicht.

4. Verfahren nach Anspruch 1, **dadurch gekennzeichnet, dass** die physikalische Gaseigenschaft ($\lambda$) der Massenfluss ($\rho \cdot v_x$) eines Gasgemisches ist, womit die durch den mikrothermischen Sensor bestimmte Grösse $\tilde{\gamma}$ das Verhältnis zwischen Wärmekapazität und -leitfähigkeit eines Gasgemisches ist, was zusammen mit Kenntnis der Wärmeleitfähigkeit ($\lambda$) die Bestimmung der Wärmekapazität (cp) erlaubt, anhand derer der Brennwert korreliert werden kann.

5. Verfahren nach Anspruch 4, **dadurch gekennzeichnet, dass** die physikalische Gaseigenschaft $\tilde{\gamma}$ zusätzlich der Volumenfluss eines Gasgemisches ist, womit aus dem Verhältnis von Massen- zu Volumenfluss zusätzlich die Dichte ($\rho$) bekannt ist, was zusammen mit Kenntnis des Brennwerts die Bestimmung des Wobbe-Index erlaubt.

6. Verfahren nach Anspruch 1, **dadurch gekennzeichnet, dass** die physikalische Gaseigenschaft ($\gamma$) die Dichte ($\rho$) eines Gasgemisches ist, womit die durch den mikrothermischen Sensor bestimmte Grösse $\tilde{\gamma}$ das Produkt aus dem Verhältnis zwischen Wärmekapazität und -leitfähigkeit mit dem Volumenfluss eines Gasgemisches ist, was zusammen mit Kenntnis der Wärmeleitfähigkeit ($\lambda$) die Bestimmung des "Wärmekapazitätsflusses" erlaubt, anhand dessen der Energiefluss korreliert werden kann.

7. Sensor zur Bestimmung einer brenntechnisch relevanten Größe eines Gasgemisches, nach dem Verfahren gemäß einem der Ansprüche 1 bis 6 arbeitend, mit einem ersten Gaseigenschaftssensor in Kombination mit einem mikrothermischen Hitzdrahtanemometer, von welchen mindestens letzteres mit dem Gasgemischfluss beaufschlagt wird, und welche nebst der vom mikrothermischen Hitzdrahtanemometer gemessenen Wärmeleitfähigkeit ($\lambda$) zueinander komplementäre Gaseigenschaften bestimmen, wobei sich die Komplementarität auf das Verhältnis ($v_x \cdot (\rho \cdot c_p/\lambda)$) zwischen Fliessgeschwindigkeit ($v_x$) und Wärmediffusivität ($\lambda/(\rho \cdot c_p)$) des Gasgemisches bezieht und sich die Wahl des ersten Gaseigenschaftssensors je nach angestrebter Korrelation zu einer brenntechnisch relevanten Größe wie Brennwert, Wobbe-Index, Methanzahl und/oder Luftbedarf ergibt.

8. Sensor nach Anspruch 7, wobei das mikrothermische Hitzdrahtanemometer ein integriertes CMOS-Hitzdrahtanemometer ist.

9. Sensor nach einem der Ansprüche 7 und 8 zur Bestimmung einer brenntechnisch relevanten Größe eines Gasgemisches mit einem Sensorblock (2) mit einem ersten Gaseigenschaftssensor und einem Sensorblock (3) mit mikrothermischen Hitzdrahtanemometer oder einem gemeinsamen Sensorblock (4) mit beiden Sensoren, welche direkt in der Gashauptleitung 1 montiert sind.

10. Sensor nach einem der Ansprüche 7 und 8 zur Bestimmung einer brenntechnisch relevanten Größe eines Gasgemisches mit einem Sensorblock (2) mit einem ersten Gaseigenschaftssensor und einem Sensorblock (3) mit mikrothermischen Hitzdrahtanemometer oder einem gemeinsamen Sensorblock (4) mit beiden Sensoren, welche an eine zu einer Gashauptleitung (1) führenden Messleitung (5) angeschlossen sind und an ihrem Gas-Auslass mit einem Absperrventil (6) verbunden sind, wobei das Absperrventil (6) zur Messung und zur Spülung des Sensors

7

geöffnet werden kann.

11. Sensor nach einem der Ansprüche 7 bis 10 mit einem ersten Gaseigenschaftssensor zur Bestimmung des Volumenflusses und einem mikrothermischen Hitzdrahtanemometer zur Bestimmung der Wärmediffusivität ($\lambda/(\rho \cdot c_p)$) und der Wärmeleitfähigkeit ($\lambda$) eines Gasgemisches zur Ausgabe der Gasart H- oder L-Gas.

12. Sensor nach Anspruch 11, wobei der Volumenfluss durch Erzeugung einer gasunabhängigen Strömungsdynamik (Taylor-Couette-Strömung) vorgegeben wird.

13. Sensor nach einem der Ansprüche 7 bis 10 mit einem ersten Gaseigenschaftssensor zur Bestimmung des Massenflusses ($\rho \cdot v_x$) und einem mikrothermischen Hitzdrahtanemometer zur Bestimmung des Verhältnisses zwischen Wärmekapazität und -leitfähigkeit und, zusammen mit der vom mikrothermischen Hitzdrahtanemometer bestimmten Wärmeleitfähigkeit ($\lambda$), des Brennwerts eines Gasgemisches.

14. Sensor nach Anspruch 13 mit einem ersten Gaseigenschaftssensor zur zusätzlichen Bestimmung des Volumenflusses und, zusammen mit der aus dem Verhältnis von Massen- zu Volumenfluss berechneten Dichte ($\rho$), des Wobbe Index eines Gasgemisches.

15. Sensor nach einem der Ansprüche 7 bis 10, mit einem ersten Gaseigenschaftssensor zur Bestimmung der Dichte ($\rho$) und einem mikrothermischen Hitzdrahtanemometer zur Bestimmung des Produkts aus dem Verhältnis zwischen Wärmekapazität und -leitfähigkeit mit dem Volumenfluss eines Gasgemisches und, zusammen mit der vom mikrothermischen Hitzdrahtanemometer bestimmten Wärmeleitfähigkeit ($\lambda$), des Energieflusses eines Gasgemisches.

**Claims**

1. Method for determining physical gas properties of gas mixtures for the correlation of combustion-relevant variables of gas mixtures by combining a first sensor for the physical gas property ($\gamma$), for example the density ($\rho$) or the heat capacity ($c_p$) or the mass or volumetric flow or a combination of these variables, with a microthermal sensor for measuring the complementary variable ($\tilde{\gamma}$) relating to the ratio ($v_x \cdot (\rho \cdot c_p/\lambda)$) between flow velocity ($v_x$) and thermal diffusivity ($\lambda/(\rho \cdot c_p)$) of the gas mixture, where $\gamma \cdot \tilde{\gamma} = v_x \cdot (\rho \cdot c_p/\lambda)$, **characterized in that** at least the microthermal sensor is subjected to a gas flow and the thermal conductivity ($\lambda$) of the gas mixture is measured separately with the microthermal sensor.

2. Method according to claim 1, **characterized in that** the physical gas property ($\gamma$) is the density, the heat capacity, the mass or volumetric flow or a combination of these variables and the combustion-relevant variable is the gross calorific value, the Wobbe Index, the methane number and/or the air requirement.

3. Method according to claim 1, **characterized in that** the physical gas property ($\gamma$) is the volumetric flow ($v_x \cdot A$) of a gas mixture, whereby the value ($\tilde{\gamma}$) determined by the microthermal sensor is the product ($(1/A) \cdot (\rho \cdot c_p/\lambda)$) of the reciprocal value ($1/A$) of the flow cross-section with the reciprocal value ($\rho \cdot c_p/\lambda$) of the thermal diffusivity of a gas mixture, which combined with knowledge of the thermal conductivity ($\lambda$) enables a differentiation into H- and L-gases.

4. Method according to claim 1, **characterized in that** the physical gas property ($\gamma$) is the mass flow ($\rho \cdot v_x$) of a gas mixture, whereby the value ($\tilde{\gamma}$) determined by the microthermal sensor is the ratio between heat capacity and thermal conductivity of a gas mixture, which combined with knowledge of the thermal conductivity allows the heat capacity ($c_p$) to be determined, on the basis of which the gross calorific value can be correlated.

5. Method according to claim 4, **characterized in that** the physical gas property ($\lambda$) is also the volumetric flow of a gas mixture, whereby the density ($\rho$) is also known from the ratio of mass to volumetric flow, which combined with knowledge of the gross calorific value allows the Wobbe Index to be determined.

6. Method according to claim 1, **characterized in that** the physical gas property ($\gamma$) is the density ($\rho$) of a gas mixture, whereby the variable ($\tilde{\gamma}$) determined by the microthermal sensor is the product of the ratio between heat capacity and thermal conductivity with the volumetric flow of a gas mixture, which combined with knowledge of the thermal conductivity ($\lambda$) allows the "heat capacity flow" to be determined, on the basis of which the energy flow can be correlated.

7. Sensor for determining a combustion-relevant variable of a gas mixture, operating in accordance with the method according to one of claims 1 to 6, with a first gas property sensor in combination with a microthermal hot-wire anemometer, of which at least the latter is subjected to the gas mixture flow, and which, in addition to the thermal conductivity ($\lambda$) measured by the microthermal hot-wire anemometer, determine complementary gas properties, where the complementarity relates to the ratio ($v_x \cdot (\rho \cdot c_p / \lambda)$) between flow velocity ($v_x$) and thermal diffusivity ($\lambda / (\rho \cdot c_p)$) of the gas mixture and the selection of the first gas property sensor depends on the desired correlation with a combustion-relevant variable such as gross calorific value, Wobbe Index, methane number and/or air requirement.

8. Sensor according to claim 7, where the microthermal hot-wire anemometer is an integrated CMOS hot-wire anemometer.

9. Sensor according to one of claims 7 and 8 for determining a combustion-relevant variable of a gas mixture with a sensor block (2) with a first gas property sensor and a sensor block (3) with microthermal hot-wire anemometer or a common sensor block (4) with both sensors, which are mounted directly in main gas line 1.

10. Sensor according to one of claims 7 and 8 for determining a combustion-relevant variable of a gas mixture with a sensor block (2) with a first gas property sensor and a sensor block (3) with microthermal hot-wire anemometer or a common sensor block (4) with both sensors, which are connected to a measuring line (5) leading to a main gas line (1) and connected to a shut-off valve (6) at their gas outlet, where the shut-off valve (6) can be opened for measuring and for flushing the sensor.

11. Sensor according to one of claims 7 to 10 with a first gas property sensor for determining the volumetric flow and a microthermal hot-wire anemometer for determining the thermal diffusivity ($\lambda / (\rho \cdot c_p)$) and thermal conductivity ($\lambda$) of a gas mixture for outputting the H- or L-gas type.

12. Sensor according to claim 11, where the volumetric flow is predetermined through generation of a gas-independent flow dynamics (Taylor-Couette flow).

13. Sensor according to one of claims 7 to 10 with a first gas property sensor for determining the mass flow ($\rho \cdot v_x$) and a microthermal hot-wire anemometer for determining the ratio between heat capacity and thermal conductivity and, together with the thermal conductivity ($\lambda$) determined by the microthermal hot-wire anemometer, the gross calorific value of a gas mixture.

14. Sensor according to claim 13 with a first gas property sensor for additional determination of the volumetric flow and, together with the density ($\rho$) calculated from the ratio of mass to volumetric flow, the Wobbe Index of a gas mixture.

15. Sensor according to one of claims 7 to 10, with a first gas property sensor for determining the density ($\rho$) and a microthermal hot-wire anemometer for determining the product of the ratio between heat capacity and thermal conductivity with the volumetric flow of a gas mixture and, together with the thermal conductivity ($\lambda$) determined by the microthermal hot-wire anemometer, the energy flow of a gas mixture.

**Revendications**

1. Procédé pour déterminer des propriétés gazeuses physiques de mélanges de gaz pour corrélation de grandeurs, importantes pour les techniques de combustion, des mélanges gazeux par combinaison d'un premier capteur pour la propriété gazeuse physique ($\gamma$), par exemple la masse volumique ($\rho$) ou la capacité calorifique ($c_p$), ou le débit massique ou volumique, ou par combinaison de ces grandeurs, avec un capteur microthermique pour la mesure de la grandeur qui lui est complémentaire ($\tilde{\gamma}$), rapportée au rapport ($v_x \cdot (\rho \cdot c_p / \lambda)$) entre la vitesse d'écoulement ($v_x$) et la diffusivité thermique ($\lambda / (\rho \cdot c_p)$) du mélange gazeux, avec $\gamma \cdot \tilde{\gamma} = v_x \cdot (\rho \cdot c_p / \lambda)$, **caractérisé en ce qu'**au moins le capteur microthermique est exposé à un écoulement gazeux, et que la conductibilité thermique ($\lambda$) du mélange gazeux est mesurée séparément avec le capteur microthermique.

2. Procédé selon la revendication 1, **caractérisé en ce que** la propriété gazeuse physique ($\gamma$) est la masse volumique, la capacité calorifique, le débit massique ou volumique, ou une combinaison de ces grandeurs, et la grandeur importante pour les techniques de combustion est le pouvoir calorifique, l'indice de Wobbe, l'indice de méthane et/ou la demande en air.

3. Procédé selon la revendication 1, **caractérisé en ce que** la propriété gazeuse physique ($\gamma$) est le débit volumique ($v_x \cdot A$) d'un mélange gazeux, la grandeur ($\tilde{\gamma}$) déterminée par le capteur microthermique étant le produit (($1/A)\cdot(\rho \cdot c_p/\lambda$)) de l'inverse ($1/A$) de la section transversale d'écoulement par l'inverse ($\rho \cdot c_p/\lambda$) de la diffusivité thermique d'un mélange gazeux, ce qui, avec la connaissance de la conductibilité thermique ($\lambda$), permet de faire une distinction entre les gaz H et les gaz L.

4. Procédé selon la revendication 1, **caractérisé en ce que** la propriété gazeuse physique ($\gamma$) est le débit massique ($\rho \cdot v_x$) d'un mélange gazeux, la grandeur ($\tilde{\gamma}$) déterminée par le capteur microthermique étant le rapport entre la capacité calorifique et la conductibilité thermique d'un mélange gazeux, ce qui, avec la connaissance de la conductibilité thermique ($\lambda$), permet de déterminer la capacité calorifique ($c_p$), à l'aide de laquelle il est possible de corréler le pouvoir calorifique.

5. Procédé selon la revendication 4, **caractérisé en ce que** la propriété gazeuse physique ($\gamma$) est en outre le débit volumique d'un mélange gazeux, grâce à laquelle, à partir du rapport entre le débit massique et le débit volumique, on connaît en outre la masse volumique ($\rho$), ce qui avec la connaissance du pouvoir calorifique permet de déterminer l'indice de Wobbe.

6. Procédé selon la revendication 1, **caractérisé en ce que** la propriété gazeuse physique ($\gamma$) est la masse volumique ($\rho$) d'un mélange gazeux, la grandeur ($\tilde{\gamma}$) déterminée par le capteur microthermique étant le produit du rapport entre la capacité calorifique et la conductibilité thermique par le débit volumique d'un mélange gazeux, ce qui avec la connaissance de la conductibilité thermique ($\lambda$) permet de déterminer le débit de capacité thermique, à l'aide duquel on peut corréler le débit énergétique.

7. Capteur pour la détermination d'une grandeur, importante pour les techniques de combustion, travaillant conformément au procédé selon l'une des revendications 1 à 6, comportant un premier capteur de propriété gazeuse en combinaison avec un anémomètre à fil chaud microthermique, dont au moins le dernier est exposé à un écoulement d'un mélange gazeux, et qui, en plus de la conductibilité thermique ($\lambda$) mesurée par l'anémomètre à fil chaud microthermique, détermine des propriétés gazeuses complémentaires l'une de l'autre, la complémentarité concernant le rapport ($v_x \cdot (\rho \cdot c_p/\lambda)$) entre la vitesse d'écoulement ($v_x$) et la diffusivité thermique ($\lambda/(\rho \cdot c_p)$) du mélange gazeux, le choix du premier capteur de propriété gazeuse étant effectué en fonction de la corrélation recherchée avec une grandeur importante pour les techniques de combustion, telle que le pouvoir calorifique, l'indice de Wobbe, l'indice de méthane et/ou la demande en air.

8. Capteur selon la revendication 7, dans lequel l'anémomètre à fil chaud microthermique est un anémomètre à fil chaud CMOS intégré.

9. Capteur selon l'une des revendications 7 et 8 pour déterminer une grandeur, importante pour les techniques de combustion, d'un mélange gazeux, comportant un bloc capteur (2) avec un premier capteur de propriété gazeuse et un bloc capteur (3) ayant un anémomètre à fil chaud microthermique ou un bloc capteur commun (4) comportant les deux capteurs, qui sont montés directement dans la conduite principale de gaz (1).

10. Capteur selon l'une des revendications 7 et 8 pour déterminer une grandeur, importante pour les techniques de combustion, d'un mélange gazeux, comportant un bloc capteur (2) avec un premier capteur de propriété gazeuse et un bloc capteur (3) avec un anémomètre à fil chaud microthermique ou un bloc capteur commun (4) avec les deux capteurs, qui sont raccordés à une conduite de mesure (5) conduisant à une conduite principale de gaz (1), et qui au niveau de leur orifice de sortie du gaz sont reliés à une vanne d'arrêt (6), la vanne d'arrêt (6) pouvant être ouverte pour la mesure et pour le rinçage du capteur.

11. Capteur selon l'une des revendications 7 à 10, comportant un premier capteur de propriété gazeuse pour déterminer le débit volumique et un anémomètre à fil chaud microthermique pour déterminer la diffusivité thermique ($\lambda/(\rho \cdot c_p)$) et la conductibilité thermique ($\lambda$) d'un mélange gazeux, pour obtenir en sortie la nature du gaz, gaz H ou gaz L.

12. Capteur selon la revendication 11, dans lequel le débit volumique est prédéfini pour produire une dynamique d'écoulement indépendante du gaz (écoulement de Taylor-Couette).

13. Capteur selon l'une des revendications 7 à 10, comprenant un premier capteur de propriété gazeuse pour déterminer le débit massique ($\rho \cdot v_x$) et un anémomètre à fil chaud microthermique pour déterminer le rapport entre la capacité calorifique et la conductibilité thermique, et, avec la conductibilité thermique ($\lambda$), déterminée par l'anémomètre à fil chaud microthermique, le pouvoir calorifique d'un mélange gazeux.

14. Capteur selon la revendication 13, comportant un premier capteur de propriété gazeuse pour la détermination supplémentaire du débit volumique et, avec la masse volumique ($\rho$), calculée par le rapport entre le débit massique et le débit volumique, de l'indice de Wobbe d'un mélange gazeux.

15. Capteur selon l'une des revendications 7 à 10, comportant un premier capteur de propriété gazeuse pour la détermination de la masse volumique (p) et un anémomètre à fil chaud microthermique pour la détermination du produit du rapport entre la capacité calorifique et la conductibilité thermique par le débit volumique d'un mélange gazeux, et, avec la conductibilité thermique ($\lambda$) déterminée par l'anémomètre à fil chaud microthermique, du débit énergétique d'un mélange gazeux.

Fig. 1

$$\left(\frac{c_p}{\lambda}\right) \cdot \left(\rho\right) \cdot \left(\mathbf{v_x}\right) \cdot \frac{d}{dx} T = \nabla^2 T + \frac{1}{\lambda} \Theta$$

$$\widetilde{\gamma} \quad \cdot \quad \gamma \cdot \frac{d}{dx} T = \nabla^2 T + \frac{1}{\lambda} \Theta$$

Mikrothermischer    Sensor 2
Sensor

**Fig. 2**

mikrothermisches CMOS-Anemometer

**Fig. 3**

**Fig. 4a**

**Fig. 4b**

**Fig. 5a**

**Fig. 5b**

**Fig. 6a**

**Fig. 6b**

**Fig. 6c**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2015056 B1
- US 7188519 B
- EP 1119744 A
- US 5311447 A
- EP 1155690 A

**In der Beschreibung aufgeführte Nicht-Patentliteratur**

- Prandtl Führer durch die Strömungslehre 10. Braunschweig/Wiesbaden. Friedr. Vieweg & Sohn, 2001
- **KERSON HUANG.** Statistical Mechanics. John Wiley & Sons, 1987
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146